Europäisches Patentamt

European Patent Office

Office européen des brevets

(11) Publication number: **0 327 801**

**A1**

# EUROPEAN PATENT APPLICATION

(21) Application number: **89100160.4**

(22) Date of filing: **05.01.89**

(51) Int. Cl.4: **C12N 7/00 , C07K 15/00 , C12N 9/88 , A61K 39/21 , C07K 3/28 , C12P 21/00 , G01N 33/569**

The applicant has filed a statement in accordance with Rule 28 (4) EPC (issue of a sample only to an expert). Accession number(s) of the deposit(s): ECACC 87122301.

(30) Priority: **08.01.88 EP 88100191**

(43) Date of publication of application:
**16.08.89 Bulletin 89/33**

(84) Designated Contracting States:
**AT BE CH DE ES FR GB IT LI NL SE**

(71) Applicant: **DEUTSCHES PRIMATENZENTRUM GESELLSCHAFT MBH**
**Kellnerweg 4**
**D-3400 Göttingen(DE)**

(72) Inventor: **Hunsmann, Gerhard, Prof.Dr.med.**
**Holundersteg 9**
**D-3400 Göttingen(DE)**
Inventor: **Schneider, Josef, Dr.rer.nat.**
**Südring 44**
**D-3406 Bovenden(DE)**
Inventor: **Lueke, Wolfgang., Dr.rer.nat.**
**Mittelstrasse 3F**
**D-3400 Göttingen(DE)**

(74) Representative: **Vossius & Partner**
**Siebertstrasse 4 P.O. Box 86 07 67**
**D-8000 München 86(DE)**

(54) HIV-2-type retroviruses of primates, vaccines, diagnostic and pharmaceutical compositions.

(57) The HIV-2-type retroviruses of primates HIV-2ben and mutants and derivatives thereof are provided. These viruses are a source for the isolation of proteins p18, p24, the reverse transcriptase,and of proteins gp130 and gp160. The viruses, their proteins, and corresponding monoclonal antibodies can be used for the preparation of vaccines, diagnostic and pharmaceutical compositions.

EP 0 327 801 A1

## HIV-2-type retroviruses of primates, vaccines, diagnostic and pharmaceutical compositions

The present invention relates to HIV-2-type retroviruses of primates. In particular it relates to the HIV-2-type retrovirus HIV-2ben and proteins p18, p24, the reverse transcriptase containing the polypeptides p54 and p66 as well as the envelope proteins gp130 and gp160 thereof. Furthermore it relates to processes which are generally applicable for the production of said proteins of retroviruses.

Still further the invention relates to monoclonal antibodies directed to said proteins, to the corresponding hybridomas, and to pharmaceutical compositions containing said monoclonal antibodies. Additionally the invention relates to the use of said proteins and monoclonal antibodies for the production of vaccines, to the use of said viruses, proteins and monoclonal antibodies for the diagnosis of infections of mammalians with HIV-2-type retroviruses, to vaccines containing said proteins or monoclonal antibodies, and to diagnostic kits containing said viruses, proteins and/or monoclonal antibodies

The infection with HIV-1 (human immunodeficiency virus type I), formerly also designated as HTLV-III, is spreading world wide almost exponentially, WHO estimates, that up to 10 million people might already have been infected. Usually the infection is latent for many years. However most of the infected individuals will probably develop symptoms of immunodeficiency and neurological disease caused by immune and nervous system infection with HIV-1. At present the spread of this infection can neither be stopped by an effective vaccine nor can this disease be treated with an effective drug generally available.

The origin of HIV-1 is still a mystery. However in recent years related viruses have been isolated from 7 Old World primate species and at least another 5 species were found to have serum antibodies that crossreact with simian immunodeficiency viruses SIV). It is worth noting, that certain African species e.g. African green monkeys (agm) and sooty mangabey are infected to approximately 50 % with SIV. However, disease symptoms similar to those related to HIV-1 infection in man have never been observed. Transmission of viruses from these species to certain macaques e. g. rhesus monkeys leads to the development of an AIDS-like disease in these animals. Thus the original African hosts of these SIVs seem to have adapted to the pathogenic potential of their respective lentiviruses (1).

The discovery of these SIVs lends credit to the hypothesis that human AIDS was caused by interspecies transmission of such a monkey virus into humans. This possibility was further strengthend by the detection of antibodies in people from West Africa and some AIDS patients crossreacting with SIV but only weakly if at all with HIV-1. A new virus called HIV-2 was isolated from such individuals (2). Analyses of such isolates showed that it is more closely related to certain SIVs than to the original human AIDS virus HIV-1 (2, 3). Infection with this virus cannot regularly be detected by the commercially available HIV-1 antibody screening test. Therefore the percentage of HIV-2 infected individuals e.g. in Africa, United States, and Europe is unknown, but preliminary data seem to indicate, that it is still low compared to the frequency of HIV-1 infections. In conclusion we are not only faced with a rapidly spreading epidemic of HIV-1 and its consequences but also with a situation in which additional types of lentiretroviruses of non-human primates probably are able to cross the species border to man via an unknown route and subsequently cause AIDS and other diseases in the non-adapted human population.

It is therefore very important to isolate such retroviruses from patients and to subsequently use them or components thereof for prophylaxis or diagnosis. In this respect it is of particular importance to isolate said retroviruses as soon as possible after they have crossed the species border since only a reliable diagnosis and the use of vaccines can effectively prevent further spreading of the viruses among human populations.

Researchers of the Pasteur Institute in Paris, France, have described four HIV-2 isolates namely HIV-2rod, HIV-2dul, HIV-2gom, and HIV-2mir (2, 3, 4). All four isolates were obtained from AIDS patients or seropositive individuals from Westafrica. These isolates belong to a new serotype of the human immunodeficiency virus distinct both from HIV-1 as well as from SIVmac, an isolate from rhesus macaques. Core proteins of HIV-1 and HIV-2 show some extent of serological cross-reactivities but their envelope polypeptides did not cross-react. Interestingly a serum from an HIV-2 infected AIDS patient cross-reacted only with the SIVmac glycoprotein, but not with the core protein. Similarly a serum from an infected macaque cross-reacted likewise with respect to the envelope but not with the core proteins (1). HIV-2rod has been sequenced completely and restriction enzyme analysis of three other HIV-2 isolates have been performed with three restriction enzymes. These results indicate that all four isolates are distinct from each other (2, 5). Dot blot hybridization experiments showed, that HIV-2rod and HIV-2dul cross-hybridize. A weaker cross-hybridization was observed with SIVmac, but no reaction was seen with an HIV-1 probe (3). Further HIV-2 isolates have obviously been obtained by the same group, but were not characterized further (3). These HIV-2 isolates only represent some members of a new group of HIV.

However, the isolation of new human retroviruses including additional HIV-2 subtypes is necessary to develop specific diagnostic reagents and vaccines, which finally cover the whole field of HIV-2 type retroviruses of primates. New isolates which may be genetically related to already known viruses often have strikingly different patterns of pathogenicity. This phenomenon is also frequently observed in other families of animal viruses. Therefore it is important to provide highly specific reagents for epidemiological studies as well as for the prognosis of the course of infections in individual patients. Such diagnostic reagents may consist of monoconal antibodies or genetic probes. In this context the obvious neurotropism of the HIV-2ben isolate of the present invention seems to be relevant.

Since newly isolated HIVs are more related to simian viruses, new retroviruses could enter the human population by transspecies-infection. Thus, specific reagents are also required to confine the infection of the human population at an early stage and to trace the route of the interspecies transmission.

HIVs are highly variable in their surface glycoproteins. Vaccines consisting of glycoproteins can block infection with animal retroviruses. However the blocking mechanism is predominantly type-specific. Potential vaccines against HIV must therefore include a broad spectrum of serotypes.

Thus it is the technical problem underlying the present invention to provide novel HIV-2-type retroviruses of primates and means for their diagnosis and for prophylaxis and treatment of infections with these retroviruses.

This technical problem is solved by providing the embodiments characterized in the claims.

In the following the invention is further explained and illustrated by the figures:

Figure 1:

Analysis of the polypeptides of various SIV, HIV-1, and HIV-2 preparations by polyacrylamide gel electrophoresis in the presence of sodium dodecylsulfate (SDS-PAGE).

SIV was produced in Molt-4; HIV-1 in H9 cells and HIV-2rod and HIV-2ben in Jurkat cells. Virus was pelleted from cell-free culture fluid as described in ref.(6)and resuspended in 0,1 % of the original volume of PBS. 2, 5 and 10 ul of the suspension were mixed with the same volume of reducing sample buffer (7). The mixtures were heated for 3 min to 96° C and applied to a conventional SDS-PAGE (7). The patterns of the Coomassie blue stained polypeptides show, that HIV-2ben is similar but distinct from HIV-2rod. HIV-2ben is produced more efficiently than HIV-1, but less efficiently than HIV-2rod.

Figure 2:

Comparison of HIV-2ben and HIV-2rod by radioimmunoprecipitation assay (RIPA)

Jurkat cells infected either with HIV-2ben or HIV-2rod were labelled with $^{35}$S-cysteine as described in ref.(7).Extracts of the labelled cells were incubated with the sera from an uninfected control person (lane 1), two sera of HIV-1 infected persons (lanes 2 and 3), a serum of an SIV infected African green monkey (lane 4) and two sera of HIV-2 infected AIDS patients (lanes 5 and 6). The immunocomplexes were purified by adsorption to protein A-sepharose and washing. The bound antigens were then analysed by SDS-PAGE. Obviously serum 2 reacts much better with the gp130 of HIV-2rod than with the glycoprotein of HIV-2ben. Sera 3 and 4 precipitate the gag precursor 55 of HIV-2ben but do not react at all with the similar polypeptide of HIV-2rod. The opposite situation is found with serum No. 5 which precipitates the smaller core polypeptide of HIV-2rod but reacts very weakly with similar polypeptides of HIV-2ben. In conclusion this serological comparison between the two viruses clearly shows, that the HIV-2ben is a distinct entity.

Figure 3:

Peptide maps of p18 and p24 of HIV-2rod and HIV-2ben.

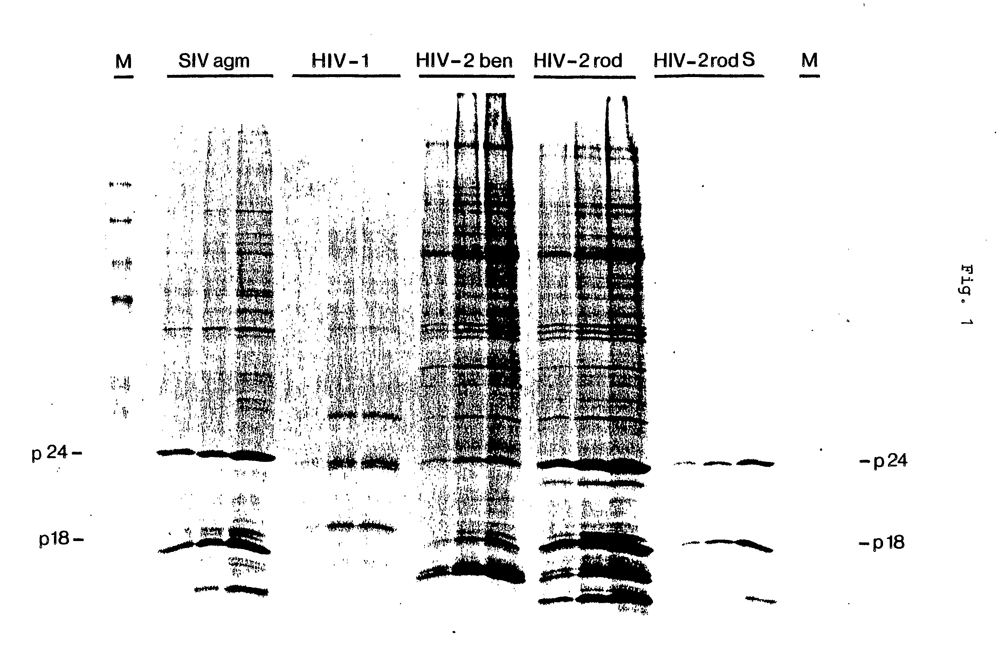

Virus of both types was propagated in Jurkat cells and isolated as described in Fig.1. Virus was solubilized and radioiodinated as described in reference (9). The viral proteins p18 and p24 were then

3

purified by immunoprecipitaion and SDS-PAGE prior to digestion with trypsin. The tryptic fragments were then analysed on cellulose-coated thin layer plates by electrophoresis in one direction and chromatography in the perpendicular direction. The figure shows autoradiographs of the peptide maps. The p18 peptide-maps of both viruses were very similar, however the pattern of p24 maps showed striking differences. At least two of the major five spots are missing in HIV-2ben and several minor spots are also not detectable in HIV-2ben p24.

Figure 4:

Different efficiencies of replication of HIV-2rod and HIV-2ben in various human T-cell lines.

Logarithmically growing cultures of the cell lines H9, Jurkat, Molt-4, MT-2, and MT-4 were diluted with RPMI-1640 medium and infected with virus from HIV-2ben or HIV-2rod infected Jurkat cells. The listed cell lines were infected with increasing volumes (5%, 20% or 50% of the original culture volume) of a cell-free culture fluid of the infected Jurkat cells. Three days after the infection the amount of virus in the infected cultures was estimated by a reverse transcriptase assay as described in reference (10). For each cell line the amount of alpha-$^{32}$P-desoxythymidinetriphosphate (in counts per minute, cpm) incorporated into DNA is plotted versus the concentration of Jurkat culture fluid used for infection. In general, HIV-2rod replicates more efficiently in these human cell lines. However, both MT cell lines produce HIV-2ben at least as good as HIV-2rod when infected with low doses of virus.

Figure 5:

The cytopathic effect of HIV-2ben to MT-4 cells exceeds that of HIV-2rod and can be used to measure virus infectivity.

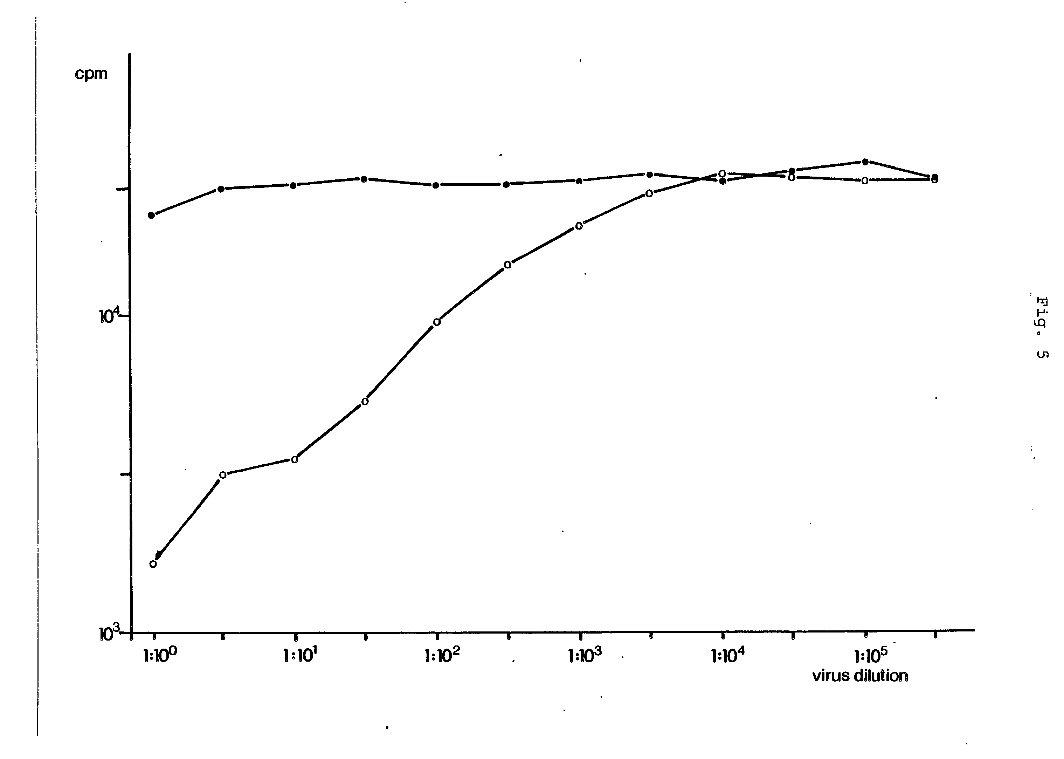

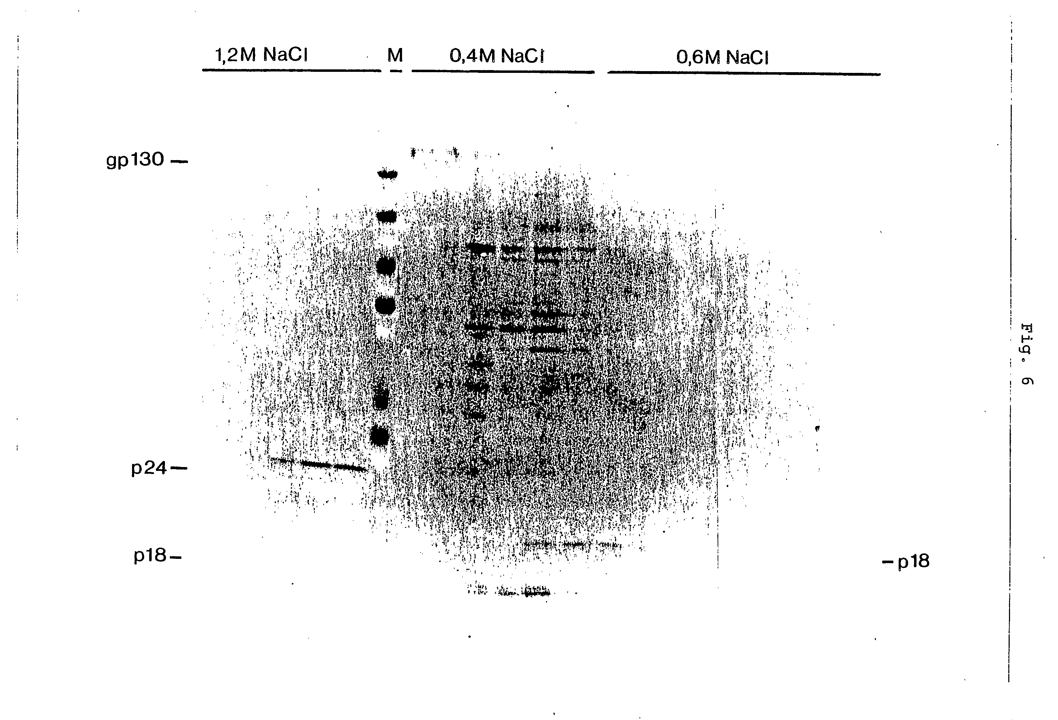

In contrast to the test described in Fig. 4, the infectivity assay measures the killing of cells by viral infection. The test has been performed according to a procedure published in ref. (11). The MT-4 cells were incubated with the virus inoculum indicated on the horizontal axis. After one week $^{3}$H-thymidine was added to the cultures. After 16h of incubation the incorporated thymidine was determined. Infection with HIV-2ben (O-----O) but not the infection with HIV-2rod (●-----●) blocks the DNA replication of MT-4 cells.
Thus we have established an infectivity assay for HIV-2ben, which can also be used to examine the virus neutralizing capacity of HIV-specific antisera or sera of infected individuals as described for HIV-1 in ref. (11).

Figure 6:

Purification of virion polypeptides

Virus was isolated from SIV-infected Molt-4 cells by three differential steps of sedimentation as described earlier (6). Pelleted virus from 5 liters of culture was resuspended in 5ml of 10mM N,N´-bis-hydroxyethyl 2-ethanesulfonate (BES), pH6.5. Virus proteins were solubilized with Triton X-100 and bound to a phosphocellulose column as described in Example 3. After elution of the bound proteins with a sodium chloride gradient the proteins of the separately collected fractions were precipitated by ethanol and analysed by SDS-PAGE. The figure shows the Coomassie blue stained gel. The salt concentrations used for the elution of the respective proteins are indicated on top of the gel.
Chromatography on phosphocellulose clearly separates the virion polypeptides gp130, p24 and p18. The procedure avoids denaturation of the proteins and therefore preserves their biological activities.
It has to be understood that this purification process is generally applicable.

Figure 7:

Isolation of gp130/gp160 and aggregation of gp130/gp160 by sedimentation of the polypeptides into a detergent-free saccharose gradient.

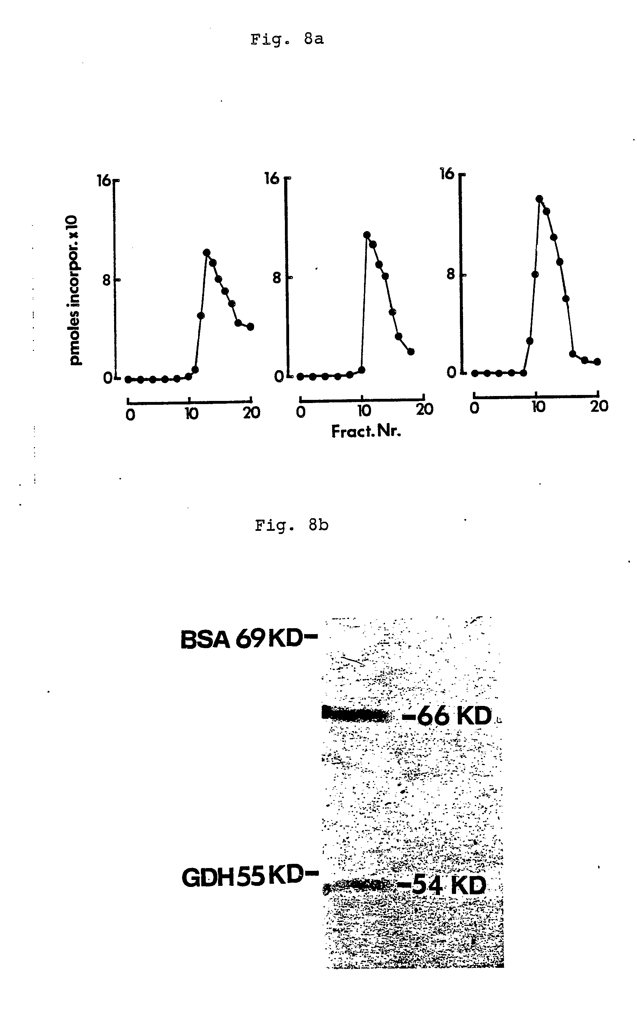

For purification of gp130/gp160 the virus was solublized with Triton X-100 as described in Fig. 6 The solubilized virus proteins were adsorbed to DEAE-cellulose and eluted with 0.3 M potassium chloride.

A) Formation of gp130/gp160 aggregates

The eluate of the DEAE-cellulose column was adsorbed to lentil-lectin sepharose and eluted with 0.4 M alpha-methylmannoside. The eluate was layered on top of a 5%-20% saccharose gradient in PBS and spun in a Kontron rotor TST 41.14 at 35 000 rpm and 4°C for 16 h. Fractions of the gradient were collected by puncturing the centrifuge tube at the bottom. Proteins were precipitated by ethanol from 20 % of the fraction volume (fraction numbers indicated on top of the gel) and analysed by SDS-PAGE. By staining of the gel with Coomassie blue it became evident that gp130 migrates faster through the gradient than polypeptides of similar size. By this sedimentation the detergent was removed and gp130 and gp160 were further purified .

B) Electronmicrograph of the gp130/gp160 aggregates.

Fractions containing the glycoproteins were pooled and dialysed against PBS to remove saccharose. Particles in this pool were visualized by adsorption to a carbon-coated formvar grid, negative-stained with uranylacetate and visualized by transmission electron microscopy. The bar in the electronmicrograph represents 100 nm.
It has to be understood that this purification process is generally applicable.

Figure 8:

Purification of reverse transcriptase (RT).

Virus was harvested from the supernatant of SIVagm-2 producing Jurkat cells as described in ref. (6). Pelleted virus from 3 l was suspended in 3 ml of 10 mM Tris x HCl buffer pH 8 containing 1% Triton X-100. This mixture was kept on ice for 30 min and thereafter centrifuged at 10 000 x g and 4 °C for 15 min. The supernatant was layered onto a 20 - 70 % saccharose in PBS pH 7.2 and sedimented (Kontron rotor TST 41.14) at 200 000 x g and 4°C for 18 hours. The gradient fractions were analysed for RT activity. Peak fractions were pooled , adjusted to 10 % saccharose in PBS pH 7.2 containig 1% Triton X-100 and sedimented on a second saccharose gradient as described above. Fractions containing maximum RT activity were adjusted to 1 % Triton X-100 and dialysed against 10 mM Tris x HCl, pH 8 containing 1 % Triton X-100 at 4°C for 16 hours. The dialysed material was kept in 1 ml aliquots at - 80°C. For further purification and characterization a 1 ml aliquot was thawed and sedimented into a 10 - 40% glycerol in 10 mM Tris x HCl pH 8.0 containing 500 mM KCl and 1mm EDTA gradient. Sedimentation conditions were described above. The RT profiles of the three gradients are shown in (A). Fractions containing maximum RT activity were pooled. This material was used for RT inhibition studies with variuos compounds. The equivalent of virus harvested from 0.5 l was ethanol precipitated and analysed by SDS-PAGE (B). This process yields about 1ug of purified enzyme per liter of cell culture.
It has to be understood that this purification process is generally applicable.

Figure 9:

Induction of a strong antibody response by immunization of rhesus monkeys with aggregated gp130/160.

Twenty microgram of gp130/gp160 aggregates mixed with or coupled to KLH with glutaraldehyde in 0.5 ml of PBS were emulsified with the same volume of incomplete Freund's adjuvant and injected intramuscularly and subcutaneously into rhesus monkeys three times in monthly intervals. Two weeks after the last injection blood was taken to search for antibodies in serum prepared thereof. An ELISA was performed as described in example 4 using gp130/gp160 as antigen and peroxidase-labelled goat anti human IgG as tracer for the rhesus antibodies. In the assay 10 ng of the gp130/gp160 preparation were used per well of the microtiter plates and the sera to be tested were serially diluted from 6,400 fold to 1.638,000 fold. In the sera of two immunized animals antibodies were clearly detectable at 200,000 fold (O----O) or 50,000 fold (●------●) dilution respectively. A monkey with a natural SIV infection developed a comparable antibody titer against the glycoprotein of SIV (■-------■). A monkey immunized with adjuvant only developed a significantly lower reactivity in this assay (x-----x). Thus gp130/gp160 aggregates induce a strong antibody response upon injection into rhesus monkeys.

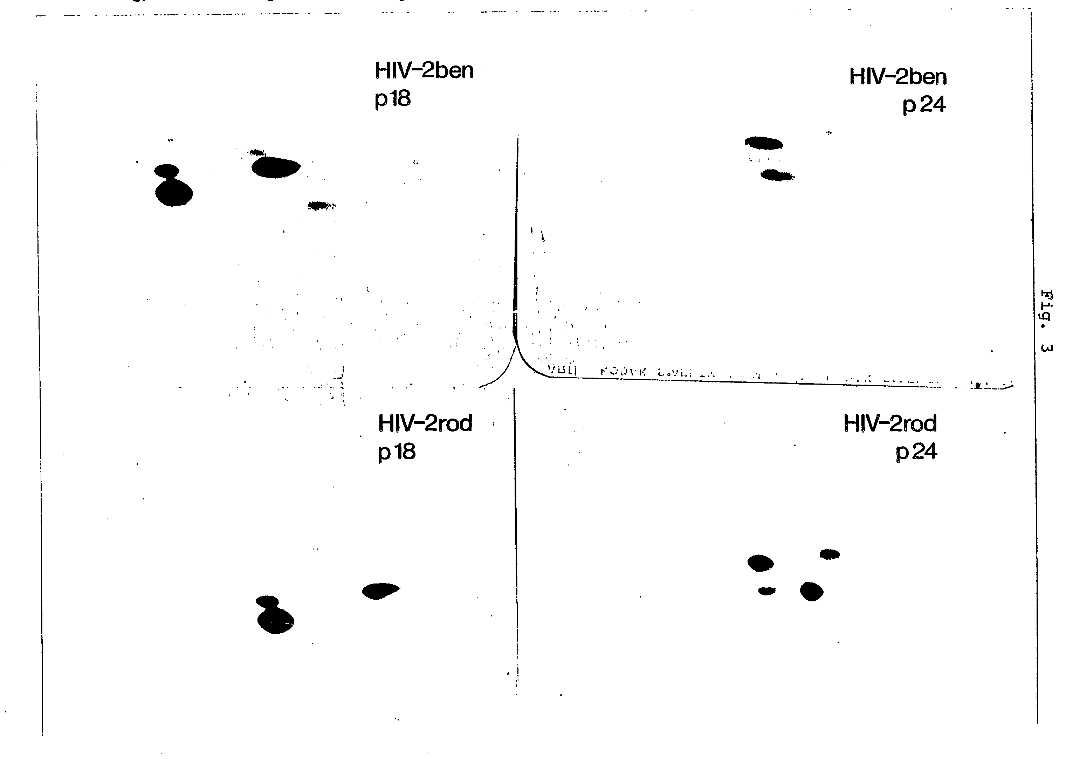

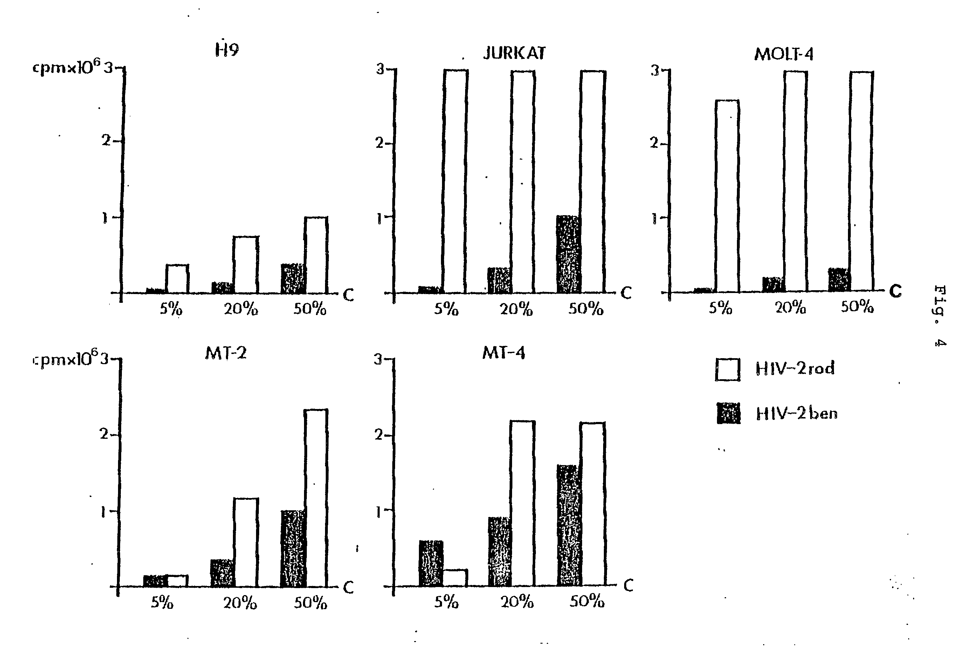

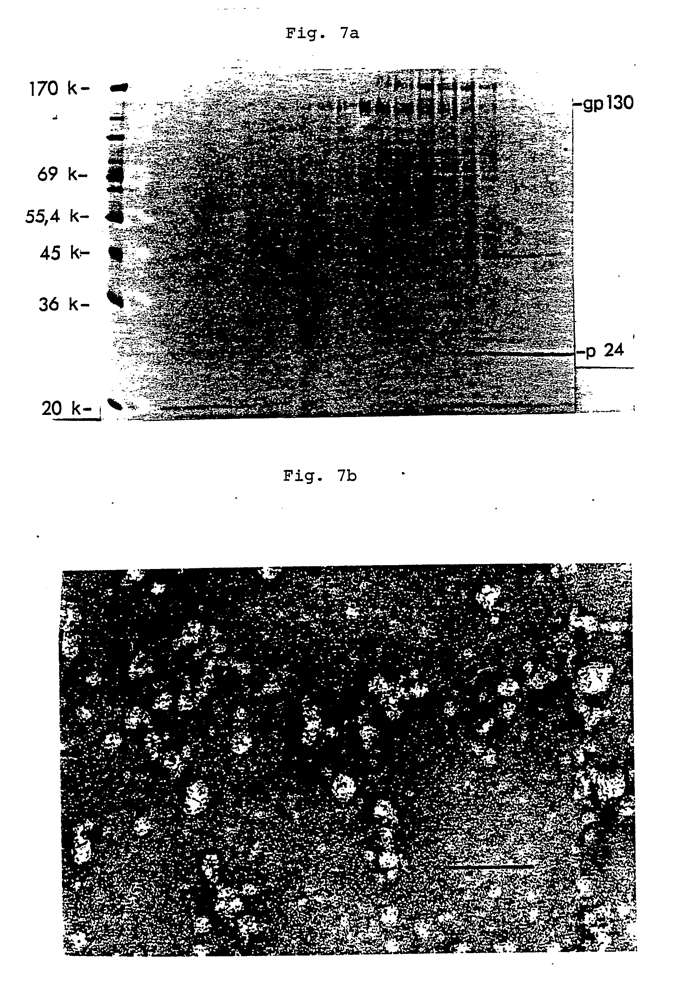

One object of the present invention is to provide the HIV-2-type retrovirus of primates HIV-2ben ECACC 87122301 displaying the following characterizing features:
-the core proteins p18 and p24 have the tryptic peptide maps shown in Fig. 3a;
-the pattern of restriction enzyme cleavage fragments of the viral genome is as described in Tab I;
-the in vitro growth characterisics are as shown in Figs. 4 and 5; and
-neurotropism in vivo as described in example 1;
and mutants and variants thereof exhibiting the same phenotypical properties. HIV-2ben was deposited under the requirements of the Budapest Treaty with the European Collection of Animal Cell Cultures (ECACC), PHLS-Center for Applied Microbiology and Research, Porton Down, Salesbury, SP40JG, U.K., on December 23, 1987 under the deposition number ECACC 87122301.

The preferred embodiment of the present invention is HIV-2ben, ECACC 87122301. This virus displays the above mentioned features. HIV-2ben was isolated from a patient with predominantly neurological symptoms and differs from the known HIV-2rod in biological properties such as its cytopathic potential and its capacity to replicate permanently in certain human T-cell lines. Furthermore HIV-2ben is structurally different from HIV-2rod; see Table I, below.

Substantial amounts of HIV-2ben are produced by permanently infected T-cell lines. This production system allows the preparation and biochemical characterization of the major envelope and core proteins gp130 and gp160, and p18 and p24, respectively, and of the two polypeptide chains of the reverse transcriptase. Said biochemical characterization and preparation is illustrated hereinafter in more detail.

A further object of the present invention is to provide proteins p18 and p24 having the tryptic peptide maps shown in Fig. 3 which are obtainable from the above mentioned viruses, preferably from HIV-2ben ECACC 87122301, by:
-treatment of a virus preparation with a non-ionic detergent;
-chromatography of the obtained dissolved virus components on phosphocellulose;
-elution of the adsorbed virus protein mixture with increasing concentrations of sodium chloride in 10 mM N,N-bis[2-hydroxyethyl]-2-aminoethanesulfonic acid (BES) (0.4M NaCl, 30 min; 0.6M NaCl, 30 min; 1.2M NaCl, 30 min) whereby gp130 and gp160 are eluted as a mixture with other contaminating proteins, while p18 and p24 are eluted in pure form and collected in separate fractions; and
-concentration of p18 and p24 by negative pressure dialysis.

Furthermore it is the object of the present invention to provide a purified, highly active viral reverse transcriptase containing the polypeptides p54 and p66 of said viruses, preferably of HIV-2ben ECACC 87122301, for enzymatic characterization as well as for inhibition studies with potential antiviral compounds. This was achieved by the process consisting of the three following steps:
-extraction of the virus with detergent,
-two cycles of sedimentation on a saccharose gradient; and
-another density gradient centrifugation on a glycerol gradient.

The preferred extraction conditions comprise suspension of the virus in 10 mM Tris x HCl buffer,pH 8,containing 1 % Triton X-100; see also legend to Fig. 8.

The preferred gradient centrifugations are sedimentation twice on a 20 - 70% saccharose in phosphate buffered saline (PBS), pH 7.2, gradient at 200 000 x g and 4°C for 18 hours followed by a single sedimentation on a 10-40% glycerol in 10 mM Tris x HCl,pH 8.0,containing 500 mM KCl and 100 mM EDTA gradient; see also legend to Fig. 8

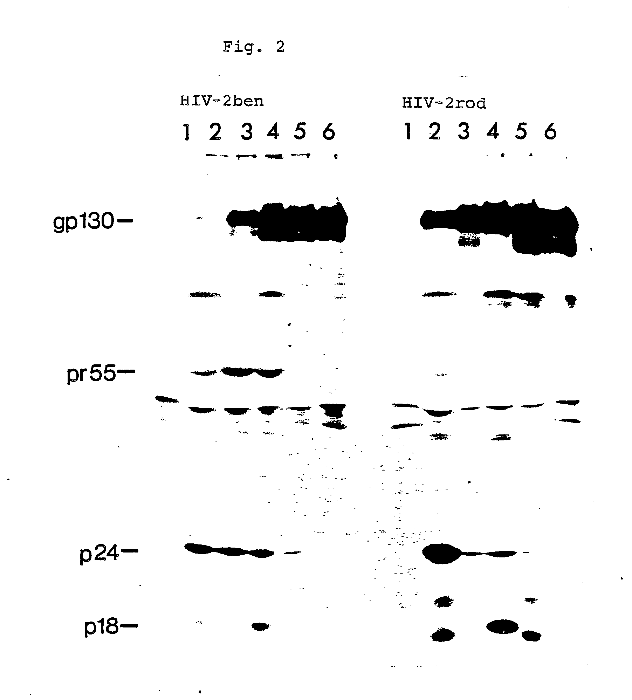

Still further it is the object of the present invention to provide the proteins gp130 and gp160 exhibiting the immunological characteristics shown in Fig. 2. These proteins are obtainable from the above mentioned viruses, preferably from HIV-ben ECACC 87122301,by:
-treatment of the virus preparation with a non-ionic detergent;
-chromatography of the obtained dissolved virus components on DEAE-cellulose;
-elution of the adsorbed virus protein mixture with 0.3 M potassium chloride as mixture with other contaminating proteins;

-purification of the eluted gp130 and gp160 by binding them to lentil lectin and eluting them with 0.4M alpha-methylmannoside in PBS; and

-separation of the non-ionic detergent and the protein by ultracentrifugation in a detergent-free saccharose gradient.

Another object of the present invention is to provide a process which is generally applicable for the production of the proteins p18 and p24 or functional equivalents thereof of retroviruses of primates which comprises the steps of:

-treatment of a virus preparation with a non-ionic detergent;

-chromatography of the obtained dissolved virus components on phosphocellulose;

-elution of the adsorbed virus protein mixture with increasing concentrations of an alkali metal salt such as KCl or NaCl in a suitable buffer having a pH of about 6.5, preferably of sodium chloride in 10mM BES,pH 6.5, (0.4M NaCl, 30 min;0.6M NaCl, 30min; 1.2M NaCl, 30 min), whereby gp130 and gp160 are eluted as mixture with other contaminating proteins, while p18 and p24 are eluted in pure form and collected in separate fractions; and

-concentration of p18 and p24 by negative pressure dialysis. The proteins obtainable according to this process are highly pure.

An additional object of the present invention is to provide a process for the purification of native, highly active retroviral reverse transcriptase (RT) containing the polypeptides p54 and p66 or functional equivalents thereof. The process comprises the following steps:

-extraction of the virus with detergent;

-two cycles of sedimentation on a saccharose density gradient; and

-another density gradient centrifugation on a glycerol gradient.

The preferred extraction condition comprise suspension of the virus in 10 mM Tris x HCl buffer, pH 8,containing 1 % Triton X-100; see also legend to Fig. 8.

The preferred gradient centrifugations are sedimentation twice on a 20 - 70 % saccharose in PBS,pH 7.2,gradient at 200 000 x g and 4°C for 18 hours followed by a single sedimentation on a 10 -40 % glycerol in 10 mM Tris x HCl pH 8.0 containing 500 mM KCl and 100 mM EDTA gradient; see also legend to Fig. 8.

This process is generally applicable to primate lentiviruses. The RT proteins obtainable by this process are highly pure and enzymatically active.

A further object of the present invention is to provide a process which is generally applicable for the production of proteins gp130 and gp160 of retroviruses or functional equivalents thereof which comprises the steps of:

-treatment of the virus preparation with a non-ionic detergent;

-chromatography of the obtained dissolved virus components on an anion exchange resin such as DEAE-cellulose;

-elution of the adsorbed virus protein mixture with a solution of monovalent cations, preferably with 0.3M KCl in 10mM Tris x HCl,pH 8.0, containing a non-ionic detergent, preferably 1 % Triton X-100;

-purification of the eluted gp130 and gp160 by binding them to lentil lectin and eluting them with 0.2-0.4M, preferably with 0.4M alpha-methylmannoside in a buffer, such as phosphate buffered saline (PBS), and preferably in 10 mM Tris x HCl, pH 8.0, 100 m KCl,1 mM Ca-chloride, 1mM Mn-chloride, in the presence of a nonionic detergent, preferably of 1% Triton X-100, and separation of the non-ionic detergent and the protein by ultracentrifugation in a detergent-free saccharose gradient. The proteins obtainable according to this process are highly pure.

Still further it is an object of the present invention to use the above proteins gp130, gp160, p18 or p24, the reverse transcriptase, or fragments or combinations thereof for the production of a vaccine. Proteins gp130, and gp160, p18 and p24 are preferred. Particularly preferred are proteins gp130 and gp160. This is explained in more detail in Fig. 8. From these experiments it is evident that said proteins are highly immunogenic in rhesus monkeys and thus represent the essential components of an effective vaccine.

Another object of the present invention is to provide human or murine monoclonal antibodies directed to the above mentioned viruses and/ or to the above mentioned proteins. Human monoclonal antibodies are preferred.

Furthermore, it is an object of the present invention to provide hybridomas or cloned immortalized B-cell lines producing said monoclonal antibodies. The above mentioned monoclonal antibodies can be obtained according to conventional methods.

The production of said murine monoclonal antibodies can be performed by ascites production following standard techniques (11). Typically mice are pretreated twice with 0,1 ml Pristane i.p. and then receive $10^6$

7

to 10⁸ hybridoma cells. After 2 to 3 weeks ascites develops and is taken out by syringes. Ascites can be stored in a frozen state. Alternatively the mass production of monoclonal antibodies can be performed in cell cultures, the supernatants of which are harvested. Alternatively human monoclonal antibodies can be harvested from cloned, immortalized B-cell lines.

In a further embodiment of the present invention the above mentioned viruses, proteins, fragments or combinations of said proteins, and the above mentioned monoclonal antibodies are used for the diagnosis of infections of mammalians and particularly of primates with HIV-2-type retroviruses. The diagnosis can be carried out according to known methods, such as ELISA (enzyme linked immunosorbent assay), Western blot, immunoprecipitation, or immunofluorescence.

Another object of the present invention is to provide vaccines containing the above mentioned proteins p18, p24, gp130 and /or gp160, the reverse transcriptase, or fragments or a combination thereof, or said human monoclonal antibodies and optionally pharmaceutically acceptable carriers and adjuvants. Vaccines containing proteins p18, p24, gp130, and gp160 are preferred. Particularly preferred are vaccines containing proteins gp130 and gp160. Per injection about 1 to 100 ug are used of said viral proteins. Typically 20 ug are administered per injection.

In a preferred embodiment of said vaccines the protein p18, p24, gp130, gp160 or the combinations thereof are contained in an aggregated form.

Said vaccines containing human monoclonal antibodies are generally designated as antiidiotypic vaccines.

Another object of the present invention is to provide diagnostic kits comprising the above mentioned virus, protein, fragments or combinations of said proteins, and/ or monoclonal antibody. Preferably preparations of the envelope proteins gp130 and gp160 are contained in said kits. Particularly preferred are embodiments of the diagnostic kits in which the envelope proteins are contained in an aggregated form.

Finally it is an object of the present invention to provide pharmaceutical compositions for the therapy of HIV-2-type infections of primates comprising the above mentioned monoclonal antibodies and optionally pharmaceutically acceptable carriers and adjuvants. The compositions either contain a single type of the above mentioned monoclonal antibodies or they contain several different types of them. As already mentioned above such monoclonal antibodies can also be used for the production of antiidiotypic vaccines. Preferably said pharmaceutical compositions contain human monoclonal antibodies.

Neutralization experiments with HIV-1 and sera from HIV-1 infected humans have shown that the prognosis of the infection is directly related to the titer of virus neutralizing antibodies (11). Furthermore antibody dependent cellular cytotoxicity has been demonstrated by others with sera from HIV-1 infected humans. Therefore one can expect that HIV-antibodies are an effective component of the anti-HIV immuneresponse in vivo.

Preferably 10 to 100mg of specific antibodies are parenterally applied, preferably by i.v. injection.

Human monoclonal antibodies have advantages for immunotherapy over murine monoclonal antibodies, since they themselves are of low immunogenicity and therefore have a much longer half-life and lower side effects, than equivalent murine monoclonal antibodies.

The invention is further illustrated by the examples

Example 1: Isolation of HIV-2ben

HIV-2ben was isolated from a 42 year old white male German citizen in September 1987. This man has been working between 1975 and 1984 in Mali, Libya and Saudi Arabia. Risk factors for HIV-1 infection were either absent or denied by the patient. The patient displayed neurological symptoms for the first time in 1984 when he developed a Raynaud phenomenon of the hands when exposed to coldness. In April 1986 he suffered from pneumonia and a single grand mal seizure. Later in 1986, he also developed an adynamia, weightloss, disturbance of concentration and impaired memory. A progressive paresis of the legs and finally incontinence occured late in 1986.

In March 1987 the patient appeared at the hospital in a generally reduced physical condition with progressive impairment of the motor activities of the lower extremities. The patient showed a marked slowing down of speech and psychomotor processes, impaired attention and concentration. A striking feature was his dark brown skin color which he noticed since he had stayed in Africa. Morbus Addison could be ruled out. Since April 1987 he developed complete paraplegia of the legs. Since about August 1987 there have been distal paraesthesias in the hand and a hyperpathia. In September 1987 contact of this skin with only lukewarm water caused redness and even blisters.

Repeated examinations of the cerebrospinal fluid showed a lymphocytic pleocytosis of between 52 and 12 cells per mm³ and the cerebrospinal fluid protein was up to values between 88 and 96 mg %. The gamma globuline fraction was markedly elevated in the cerebrospinal fluid. Computerized tomography and nuclear magnetic resonance tomography revealed an enlargement of the outer and inner fluid cavities. The EEG was unremarkable.

In March and April 1987 no HIV antibodies could be detected in several tests in the serum as well as in the cerebrospinal fluid. In July 1987 a serum sample reacted weakly in the western-blot with p24 only while the HIV-1 immunofluorescence assay remained negative. Analysis of the lymphocyte subpopulations in autumn 1987 showed a decreased T-helper /-suppressor cell ratio of 0.3. All other serological tests for viruses, bacteria, fungi and parasites were negative as were further symptoms of AIDS in particular lymphadenopathy and opportunistic infections.

In September 1987 a serological cross reactivity of the patients serum with an SIV antigen was observed in the immunoprecipitation assay (RIPA). One week after this finding virus isolation was attempted. Ten ml of the patient's blood were drawn into a heparinized syringe. Lymphocytes were isolated by the Ficoll-paque technique, suspended in culture medium RPMI 1640 suplemented with 10 % heat inactivated fetal calf serum .

To stimulate cell growth 1 % of phytohemagglutinin was added to the culture. Two days later cells were washed and mixed with unstimulated peripheral human blood lymphocytes of a healthy donor. This culture was kept for 3 weeks in standard RPMI 1640 medium plus 10 % FCS plus 10 % interleukin 2 (Lymphocult Biotest, Frankfurt). RT activity in one ml of the culture was monitored twice a week by a previously described method (9). Fresh lymphocytes of healthy donors were isolated as described above and added to the culture in intervals of ten days. After four weeks the RT activities of the particulate material from the culture supernatant increased three times over the background activities.

For infection of permanently growing human T-cell lines with HIV-2ben of the above cultures was further propagated by addition of PBLs from 100 ml of blood obtained from a healthy donor to 20 ml of RT positive culture. One week after the addition of these PBLs a high RT-activity was again measured in the culture. Twenty ml of this culture were mixed in parallel with 20ml of cultures of Jurkat, Molt-4, H-9, MT-2 and MT-4 cell lines. One week after infection the culture of infected Jurkat cells attained the highest RT-activity. Moreover the Molt-4 and the Jurkat cell lines produced RT activity for several weeks continuously. The RT activity could be pelleted by high speed centrifugation (Kontron Rotor A 6.14, 2 h at 4° C, 15.000 x g).

HIV-2ben has been deposited in the form of permanently infected MOLT-4 cells on the 23rd of December 1987 with the ECACC under the deposit No. 87122301.

Example 2: Genetic comparison of HIV-2ben and HIV-2rod

Comparison of the sizes of the fragments obtained by the cleavage of the HIV-2rod and HIV-2ben proviral DNAS with various restriction endonucleases:

The genetic difference between HIV-2ben and HIV-2rod was directly established by cleavage of their respective proviral genomes with five restriction endonucleases. DNA was prepared from Jurkat cells infected with each of the viruses, completely digested with the endonucleases, analyzed by electrophoresis in an agarose flat gel and transferred to a nitrocellulose filter. 32P-nick-translated DNA from a cloned probe consisting of the gag and pol genes of HIV-2rod was hybridized with the DNA on the nitrocellulose filter. The sizes of the fragments detected by the probes are listed. The cleavage sites of HIV-2ben differ significantly from those of HIV-2rod, yielding direct evidence for the genetic difference between the viruses as shown in Table I.

Table I

| Enzyme | HIV-2rod | HIV-2ben |
|--------|----------|----------|
|        | Size of Fragments | |
| KpnI | 4700-43900 | 5100-5300 |
| BamHI | 4900, 1700 | 5100, 2300 |
| PvuII | 6100, 4900, 1200 | 4400-4600 |
| PstI | 4200-4500, 2100 | 7000, 2400 |
| HindIII | 4200 | 7000 |
| KpnI/EcoRI | 2700, 1900 | 2700, 2300 |
| KpnI/HincII | 3600, 1100 | 3300, 1600 |
| KpnI/XhoI | 3400, 1550, 1300 | 3400, 1550 |
| KpnI/XbaI | 4550 | 5900, 5000, 2510 |
| HindIII/EcoRI | 1200, 3000 | 4700, 1150, 900 |
| HindIII/HincII | 2700, 1550 | -. |
| HindIII/XhoI | 3700 | 4600, 1800 |

Example 3: Biochemical and Biological Comparison of HIV-2ben with HIV-2 rod

Virus particles were resuspended and again pelleted as described. This pellet was resuspended in phosphate buffered saline (PBS). Aliquots of 2, 5 and 10 ul were mixed with the same volume of sample buffer, heated to 96°C for 3 min and analysed by polyacrylamide-gel electrophoresis in the SDS-PAGE (7). Fig. 1. shows the analysis of various SIV, HIV-1 and HIV-2 preparations by SDS-PAGE. SIVagm was produced in Molt-4; HIV-1 harvested from H9 cells. Both HIV-2ben and HIV-2rod were produced in Jurkat cells. The three righthand lanes show the analysis of HIV-2 purified by an additional banding in a sucrose . density gradient. Molecular weight markers comprise polypeptides of 170 kD, 94 kD, 69.5 kD, 45 kD, 36 kD, 28 and 27 kD. Since virus from an equal volume of each cell line was applied to the gel, it can be concluded, that larger amounts of HIV-2ben are produced in Jurkat cells compared to the production of HIV-1 in H9 cells. However HIV-2rod production in Jurkat cells appears to be more efficient than HIV-2ben production. The patterns of the major virus bands are distinguishable between HIV-1, HIV-2 and SIV-2. Even though HIV-2ben and HIV-2rod were produced in the same cell line there were differences in the relative intensity of various bands. This result indicates that HIV-2ben is distinct from HIV-2rod.

For further determining the differences between HIV-2ben and the Pasteur isolate viral isolates were compared by the radioimmunoprecipitation assay (RIPA). Jurkat cells infected either with HIV-2ben or HIV-2rod were labelled with $^{35}$S-cysteine (specific activity 0.5 mCi/ml) and further processed as described (7). Results of the RIPA with a negative control serum (lane 1) two anti-HIV-1 positive reference sera (lanes 2 and 3), one SIV reactive monkey serum (lane 4), and two HIV-2 specific human sera (lanes 5 and 6 ) are shown in figure 2. It is obvious that identical sera react quite differently both in quantitative and qualitative terms with individual viral polypeptides from HIV-2ben and the Pasteur isolate.

For example serum 2 reacts much better with the gp130 of HIV-2rod than with the glycoprotein of HIV-2ben. Sera 3 and 4 precipitate the gag precusor 55 of HIV-2ben but do not react at all with the similar polypeptide of HIV-2rod. The opposite situation is found with serum No. 5 which precipitates the smaller core polypeptide of HIV-2rod but reacts very weakly with similar polypeptides of HIV-2ben. In conclusion this serological comparison between the two viruses clearly shows, that the HIV-2ben is a distinct entity.

This finding was further confirmed by tryptic peptide mapping analysis of the major core polypeptides p24 and p18 of both viruses (Fig. 3). The individual polypeptides were prepared by immunoprecipitation with component specific rabbit antisera from preparations of [125]iodine-labled HIV-2ben and HIV-2rod. p18 peptide maps of both viruses were very similar, however the pattern of p24 maps showed striking differences. At least two of the major five spots are missing in HIV-2ben and several minor spots are also not detectable in HIV-2 p24. Since differences in the primary aminoacid structure are reflected in results obtained by the peptide mapping procedure the DNA sequences of both viral genomes must be distinct from each other.

Direct evidence for a genetic difference of HIV-2ben and HIV-2rod was obtained by the digestion of the respective proviral genomes with five restriction endonucleases. The results are summarized in Table 1.

The individual restriction enzymes generate different patterns of fragments from the HIV-2rod and HIV-2ben genomes. Moreover, several restriction enzymes generate several extra bands with one virus but not with the other virus. These results clearly demonstrate that extensive genetic differences exist between the two viral genomes over their entire length.

These genetic differences are confirmed by results obtained with two biological assays. Multiplication of HIV-2ben and HIV-2rod was monitored in 5 different cell lines (Fig.4). The human T-cell lines H9, Jurkat, Molt-4, MT-2 and MT-4 were infected by replacement of culture supernatent of exponentially growing cells by 5, 20 and 50 % of either HIV-2ben or HIV-2rod containing cell culture supernatent from Jurkat cells. One week later the RT activity of the particles in the culture supernatants of the newly infected cells was examined (10). In general, HIV-2rod replicates much more efficiently in these human cell lines. However, both MT cell lines produce HIV-2ben at least as good as HIV-2rod when infected with low multiplicities. Despite the more effective replication of HIV-2rod in most of the cell lines, permanently virus producing lines could not be obtained due to an inherent cytopathic effect of HIV-2rod. In contrast, HIV-2ben could be propagated to high titers in Molt-4 cells for more than 8 weeks. Thus a permanent cell line was established for HIV-2ben.

The difference in the cytopathic effect of these two viruses was further analysed in the MT-4 cell assay (Fig. 5). Serial dilutions of both viruses were added to MT-4 cell cultures in microtiter plates. After one week of culturing, $^3$H-thymidine was added to analyse cell replication. While there was almost no effect on cell replication observed after infection with HIV-2rod, HIV-2ben significantly suppressed the multiplication of MT-4 cells at high concentrations. This demonstrates, that the cytopathic potential of both viruses is remarkably different.

Example 4: Isolation of the proteins p18, p24, gp130, and gp160 contained in HIV-2ben.

Virus was purified by differential centrifugation as described in reference (6) from 6 1 of SIVagm produced in Molt-4 cells. Virus pellet was frozen at -80°C until used for preparation of the viral polypeptides.

To purify the RT pelleted virus from 3 1 was resuspended in 3 ml of 10 mM Tris X HCl buffer pH 8 containing 1% Triton X-100. This mixture was kept on ice for 30 min and thereafter centrifuged at 10 000 x g and 4°C for 15 min. The supernatant was layered onto a 20 - 70 % saccharose in PBS pH 7.2 and sedimented (Kontron rotor TST 41.14) at 135 000 x g and 4°C for 18 hours. The gradient fractions were analysed for RT activity. Peak fractions were pooled , adjusted to 1 % Triton X-100 and sedimented on a second saccharose gradient as described above. Fractions containing maximum RT activity were adjusted to 1 % Triton X-100 and dialysed against 10 mM Tris x HCl, pH 8 containing 1 % Triton X-100 at 4°C for 16 hours. The dialysed material was kept in 1 ml aliquots at -80°C. For further purification and characterization a 1 ml aliquot was thawed and sedimented into a 10 - 40% glycerol in 10 mM Tris x HCl, pH 8.0 containing 500 mM KCl and 1 mM EDTA gradient. Sedimentation conditions were described above. The RT profiles of the three gradients are shown in A. Fractions containing maximum RT activity were pooled. This material was used for RT inhibition studies with variuos compounds. The equivalent of virus harvested from 0,5 1 was ethanol precipitated and analysed by SDS-PAGE (B). This process yields about 1ug of purified enzyme per liter of cell culture. This process is generally applicable to primate lentiviruses e. g. HIV-1 and HIV-2.

To purify the core proteins p18 and p24 a virus pellet was thawed and 6 ml of 10 mM BES adjusted to pH 6.5 were added. Virus was resuspended in BES buffer by sonification for 1 minute at low energy. The protein concentration was estimated by the Bradford-assay. 200 ul of 10 % Triton X-100 in BES were added to the virus mixture. The mixtures was kept on ice for 5 minutes and then centrifuged for 8 min at 17000 g at 4°C.

The supernatant representing the virus lysate was applied onto a phosphocellulose column (Whatman P11) containing 4 ml of settled bed. The phosphocellulose had been equilibrated according to recommendations of the manufacturer with 10 mM BES. After application of the viral sample the column was washed with 50 ml of 10 mM BES without Triton. Proteins bound to the phosphocellulose were eluted with a NaCl-stepgradient in BES. The first step was an elution with 15 ml of 400 mM NaCl in BES for 30 min followed by the second step with the same volume for the same time with 600 mM NaCl and the third step for 30 min with 30 ml of 1200 mM NaCl in the BES buffer. Fractions of 2.5 ml were collected at 4°C. Individual fractions were analysed by SDS PAGE after ethanol precipitation and pelleting of precipitate for 10 min 17000g at 0°C. Protein containing fractions were adjusted to a Triton X-100 concentration of 0.5 %. Fig. 6 shows that the virion proteins gp130, p24 and p18 can be separated by phosphocellulose chromatography.

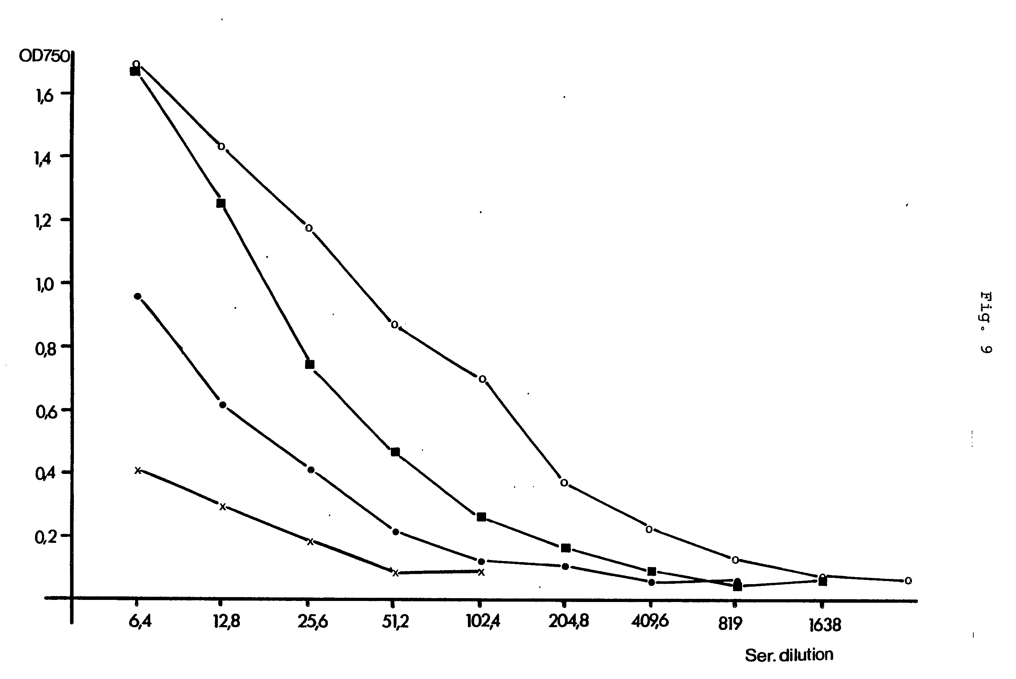

To purify the glycoprotein gp130 and gp160 pelleted virus was treated Triton X-100 as described in legend to fig. 9 and the solubilized proteins were adsorbed onto DEAE-cellulose column. Proteins were eluted with 0.3 M KCl in 10 mM Tris x HCl pH 8.0 containing 1 % Triton X-100. For furter purification the glycoproteins eluted from the DEAE-cellulose column were adsorbed to 1 ml of lentil lectin sepharose (Pharmacia). Thereafter the sepharose was washed 4 x with 10 ml of 10 mM Tris x HCl pH 8.0, 100 mM KCl, 1 mM Ca-Chloride, 1 mM Mn-Chloride, and 1 % Triton X-100. Absorbed glycoproteins were eluted with 2 ml of 400 mM alpha-methylmannoside in 10 mM Tris x HCl pH 8.0, 100 mM KCl, 1 mM Ca-Chloride, 1 mM Mn-Chloride, and 1 % Triton X-100. The eluate was layered onto the top of a 20 to 70 % sucrose density gradient in PBS. The sample was centrifuged in a Kontron rotor TST 41.14 for 18 h at 200 000 x g at 4oC. 0.7 ml fractions were collected from the gradient. The fractions were analysed by SDS-PAGE as described above.

Example 5: Isolation of the reverse transcriptase containino the polypeptides p54 and p66 of SIV, HIV-1, and HIV-2ben.

Pelleted SIVagm-2 from 3 l of culture supernatant was suspended in 3 ml of 10 mM Tris x HCl buffer pH 8 containing 1% Triton X-100, kept on ice for 30 min and centrifuged at 10 000 x g and 4 °C for 15 min. The supernatant was layered onto a 20 - 70 % saccharose in PBS pH 7.2 and sedimented at 200 000 x g and 4°C for 18 hours. Gradient fractions containing peak RT activity were pooled, adjusted to 10 % saccharose in PBS pH 7.2 containing 1% Triton X-100 and sedimented on a second saccharose gradient as described above. Fractions containing peak RT activity were adjusted to 1 % Triton X-100 and dialysed against 10 mM Tris x HCl, pH 8 containing 1 % Triton X-100 at 4°C for 16 hours. The dialysed material was kept in 1 ml aliquots at -80 °C. For further purification and characterization a 1 ml aliquot was thawed and sedimented into a 10 - 40% glycerol in 10 mM Tris x HCl pH 8.0 containing 500 mM KCl and 1mm EDTA gradient. Sedimentation conditions were described above. Fractions containing peak RT activity were pooled and used for RT inhibition studies with variuos compounds and further enzymatic analysis. This process is also applicable for purification of RTs from HIV-1 and HIV-2 and yields about 1ug of purified enzyme per liter of cell culture supernatant.

Example 6: Preparation and test of vaccine containing the glycoproteins of SIV or HIV-2ben.

Glycoprotein gp130 and gp160 containing fractions were pooled and concentrated by negative pressure dialysis against PBS in the presence of 0.2 mg/ml keyhole limpet hemocyanine (KLH). The glycoprotein solution was concentrated to about 1 ml. In order to couple the glycoprotein covalently to the KLH carrier protein, 250 ul of 20 mM glutaraldehyde in PBS was added to 1 ml of the glycoprotein solution. The reaction mixture was kept for 30 min at room temperature. To block the excess of glutaraldehyde glycin was added to a final concentration of 50 mM. The resulting solution was dialysed overnight at 4°C agains PBS then dialysed under negative pressure to a final volume of 1 ml.

Three 1,5 year-old male rhesus monkeys were immunized 3 times 4 weeks apart by intramuscular and subcutaneous injection of a SIV-glycoproteine vaccine. The vaccine was prepared by emulsification of 36 ug purified glycoprotein coupled with glutardialdehyde to KLH in incomplete Freund's adjuvant.

Serum was obtained by venipuncture from the animals before and two weeks after each immunization. Serum was prepared and examined for antibodies to gp130 by RIPA (Fig. 8). As an antigen preparation the extract of $^{35}$S-cysteine labelled SIVagm infected Molt-4 cells was used. Specific reactivity of the hyperimmune sera was only detected with gp160 and gp130 while other control sera from monkeys injected with whole inactivated virus detected the gag and pol polypeptides in addition to the glycoproteins.

Example 7: Diagnosis of SIV and HIV-2 infection bv ELISAs using the pure envelope glycoproteins of these viruses.

Sera were then titrated in a microtiter ELISA-test for antibodies to the immunising antigen. For this purpose the wells of microtiter plates were coated with 10 ng of purified SIV glycoprotein in 100 ul of coating buffer followed by incubation at 4°C over night and further incubation for 2 h at 37°C. To remove unbound protein microtiter plates were washed 3 times with PBS containing 0,05% Tween 20. To block unspecific binding sites 100 ul of PBS containing 10 % heat inactivated (30 min 56° C) newborn calf serum were added to each well and incubated for 30 min at 37 °C. Thereafter plates were washed 3 times in PBS adjusted to 0,05% Tween 20. 50 ul of serial dilutions of the respective monkey sera were then added to the wells. Dilutions were made in PBS containing 10% heat inactivated newborn calf serum and 0,05% Tween 20. Sera were allowed to react with the antigen for 1 h at 37° C. Thereafter nonbinding antibodies were washed 3 times as described above. As a second antibody goat anti-human IgG coupled to horseradish peroxidase was deluted 1/1000 in PBS plus 0,05% Tween 20, 50 ul of this second antibody dilution were added per well. After addition of the second antibody plates were kept for 30 min 37° C. Unbound second antibody was removed by washing 3 times as described above. As a substrate of the peroxidase 50 ul of PBS containing 20ug of orthophenylenediamin and 4 mM hydrogen peroxide were added. The reaction proceeded for 5 min at room-temperature and was stopped by adding 50 ul per well of 1,3 M sulfuric acid. The optical density in each well was measured by a Flow Titertek photometer at 498 nm. The optical densities obtained with the sera of the 3 immunized rhesus monkeys after the third immunisation are recorded on figure 9.

Obviously the ELISA described above can be used to examine the sera of potentially infected primates for antibodies to the respective viruses.

REFERENCES

1. Schneider J., and Hunsmann G. AIDS, 1988 in press.
2. Clavel et al., Science 233, 343, 1986
3. Clavel et al., Nature 324, 691, 1986
4. Clavel et al., New Engl. J. Med. 316, 1180, 1987
5. Guyader et al., Nature 326, 662, 1987
6. Moennig et al., Virology 61, 100, 1974
7. Lämmli et al., Nature 227 680, 1970.
8. Schneider et al. Virology 132, 1984
9. Jurkiewicz et al., Virology 150, 291, 1986
10. Jentsch et al., J. Gen. Virol. 68, 2183, 1987
11. Wendler et al., AIDS Res. and Human Retrovir. 3,157, 1987
12. Galfre' and Milstein, Methods in Enzymol. 73, 1981 et seq.

## Claims

1. HIV-2-type retrovirus of primates HIV-2ben ECACC 87122301 displaying the following characterizing features:
-the core proteins p18 and p24 have the tryptic peptide maps shown in Fig. 3a;
-the pattern of restriction enzyme cleavage fragments of the viral genome is as described in Tab. I;
-the in vitro growth characterisics are as shown in Figs. 4 and 5; and
-neurotropism in vivo;
and mutants and variants thereof exhibiting the same phenotypical properties.

2. Proteins p18 and p24 having the tryptic peptide maps shown in Fig. 3a, obtainable from a virus according to claim 1 by:
-treatment of a virus preparation with a non-ionic detergent;
-chromatography of the obtained dissolved virus components on phosphocellulose;
-elution of the adsorbed virus protein mixture with increasing concentrations of sodium chloride in 10 mM BES (0.4M NaCl, 30 min; 0.6M NaCl, 30 min; 1.2M NaCl, 30 min) whereby gp130 and gp160 are eluted as mixture with other contaminating proteins, while p18 and p24 are eluted in pure form and collected in separate fractions; and
-concentration of p18 and p24 by negative pressure dialysis.

3. Reverse transcriptase containing the polypeptides p54 and p66, obtainable from a virus according to claim 1 by:
-extraction of the virus with detergent,
-two cycles of sedimentation on a saccharose gradient; and
-another density gradient centrifugation on a glycerol gradient.

4. Proteins gp130 and gp160, exhibiting the immunological characteristics shown in Fig. 2 obtainable from a virus according to claim 1 by:

-treatment of a virus preparation with a non-ionic detergent;

-chromatography of the obtained dissolved virus components on DEAE-cellulose;

-elution of the adsorbed virus protein mixture with 0.3 M KCl in 10 mM Tris x HCl pH 8.0 containing 1 % Triton X-100;

-purification of the eluted gp130 and gp160 by binding them to lentil lectin and subsequent elution with 0.4M alpha-methylmannoside in 10 mM Tris x HCl pH 8.0, 100 mM KCl, 1 mM Ca-Chloride, 1 mM Mn-Chloride, and 1 % Triton X-100;

-separation of the non-ionic detergent and the protein by ultracentrifugation in a detergent-free saccharose gradient.

5. Process for the production of proteins p18 and p24 of retroviruses, comprising the following steps:

-treatment of a virus preparation with a non-ionic detergent;

-chromatography of the obtained dissolved virus components on phophocellulose;

-elution of the adsorbed virus protein mixture with increasing concentrations of an alkali metal salt such as KCl or NaCl in a suitable buffer having a pH of about 6.5, preferably of sodium chloride in 10 mM BES (0.4M NaCl, 30 min; 0.6M NaCl, 30 min; 1.2M NaCl, 30 min) whereby gp130 and gp160 are eluted as mixture with other contaminating proteins, while p18 and p24 are eluted in pure form and collected in separate fractions; and

-concentration of p18 and p24 by negative pressure dialysis.

6. Process for the production of retroviral reverse transcriptase comprising the following steps:

-extraction of the virus with detergent,

-two cycles of sedimentation on a saccharose gradient;and

-another density gradient centrifugation on a glycerol gradient.

7. Process for the production of gp130 and gp160 of retroviruses comprising the following steps:

-treatment of a virus preparation with a non-ionic detergent;

-chromatography of the obtained dissolved virus components on an anion exchange resin;

-elution of the adsorbed virus protein mixture with a solution containing monovalent cations and a non-ionic detergent;

-purification of the eluted gp130 and gp160 by binding them to lentil lectin and subsequent elution with alpha- methylmannoside in a buffer in the presence of a non-ionic detergent; and

-separation of the nonionic detergent and the protein by ultracentrifugation in a detergent-free saccharose gradient.

8.The use of proteins p18, p24,the reverse transcriptase, gp130, or gp160 of anyone of claims 2 to 4 or of fragments or a combination thereof for the production of a vaccine.

9. A monoclonal antibody, preferably a murine or human antibody, directed to a virus of claim 1 and/ or to a protein according to anyone of claims 2 to 4.

10. A hybridoma or an immortalized B-cell line producing a monoclonal antibody according to claim 9.

11. The use of a virus of claim 1, of a protein according to anyone of claims 2 to 4, or of fragments or a combination of said proteins, or of a monoclonal antibody according to claim 9 for the diagnosis of infections of mammalians, preferably of primates, with HIV-2-type retroviruses of primates.

12. A vaccine containing proteins p18, p24, the reverse transcriptase, gp130 or gp160 of anyone of claims 2 to 4, or fragments or a combination thereof, or a human monoclonal antibody according to claim 9, and optionally pharmaceutically acceptable carriers and adjuvants.

13. The vaccine according to claim 12, in which the protein p18, p24, the reverse transcriptase, gp130 or gp160 or the combination thereof is contained in aggregated form.

14. Diagnostic kit comprising an inactivated virus of claim 1, a protein according to anyone of claims 2 to 4, fragments or a combination of said proteins and/ or a monoclonal antibody according to claim 9.

15. Pharmaceutical compositions for the therapy of HIV-2-type infections of primates comprising a monoclonal antibody according to claim 9, preferably a human monoclonal antibody, and optionally pharmaceutically acceptable carriers and adjuvants.

Fig. 1

Fig. 2

Fig. 3

Fig. 4

Fig. 5

Fig. 6

Fig. 7a

170 k –

69 k –

55,4 k –

45 k –

36 k –

20 k –

– gp 130

– p 24

Fig. 7b

Fig. 8a

Fig. 8b

Fig. 9

European Patent Office

EUROPEAN SEARCH REPORT

Application Number

EP 89 10 0160

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 239 425 (INSTITUT PASTEUR) * Whole document * | 1,2,4,7 -15 | C 12 N 7/00 C 07 K 15/00 C 12 N 9/88 A 61 K 39/21 C 07 K 3/28 C 12 P 21/00 G 01 N 33/569 |
| X | THE LANCET, vol. 1, 17th January 1987, pages 128-132; F. BRUN-VEZINET et al.: "Lymphadenopathy-associated virus type 2 in AIDS and AIDS-related complex" * Whole document, especially page 131, column 2, lines 38-44 * | 1 | |
| P,X | EP-A-0 269 520 (INSTITUT PASTEUR) * Whole document, especially page 15, lines 5,6 * | 1,2,4,7 -15 | |
| X,D | NATURE, vol. 324, 18th-24th December 1986, pages 691-695; F. CLAVEL et al.: "Molecular cloning and polymorphism of the human immune deficiency virus type 2" * Whole document, especially page 694, column 1, lines 23-31 * | 1 | |
| X | WO-A-8 702 892 (PRESIDENT AND FELLOWS OF HARVARD COLLEGE) * Page 9, lines 2-5 * | 4,7 | TECHNICAL FIELDS SEARCHED (Int. Cl.4) C 12 N A 61 K G 01 N |
| X | PROC. NATL. SCI. USA, vol. 82, May 1985, pages 3481-3484; M.G. SARNGADHARAN et al.: "Immunological properties of the gag protein p24 of the acquired immunodeficiency syndrome retrovirus (human T-cell leukemia virus type III)" * Page 3481, column 2, lines 17-42 * | 2,5 | |
| P,X | EP-A-0 283 327 (INSTITUT PASTEUR) * Claims * ---- -/- | 11-14 | |

The present search report has been drawn up for all claims

| Place of search | Date of completion of the search | Examiner |
|---|---|---|
| THE HAGUE | 25-04-1989 | SKELLY J.M. |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | SCIENCE, vol. 231, 14th March 1986, page 1289; FULVIA DI MARZO VERONESE et al.: "Characterization of highly immunogenic p66/p51 as the reverse transcriptase of HTLV-III/LAV" * Whole document * | 3,11-14 | |
| A | THE LANCET, vol. 2, no. 8508, 2nd September 1986, page 660, London, GB; B. ASJO et al.: "Replicative capacity of human immunodeficiency virus from patients with varying severity of HIV infection" | | |
| A | NATURE, vol. 328, 6th August 1987, pages 543-547; L. CHAKRABARTI et al.: "Sequence of simian immunodeficiency virus from macaque and its relationship to other human and simian retroviruses" * Page 547, column 2, lines 4-20 * | 1 | |
| P,X | ZEITSCHRIFT FÜR NATURFORSCHUNG, vol. 43, no. 5/6, section C, Biosciences, 1988, pages 449-454, Tübingen, DE; E. JURKIEWICZ et al.: "Serological and structural comparison of immunodeficiency viruses from man, African green monkey, rhesus monkey and sooty mangabey" * Whole document * | 1-15 | TECHNICAL FIELDS SEARCHED (Int. Cl.4) |

The present search report has been drawn up for all claims

| Place of search | Date of completion of the search | Examiner |
|---|---|---|
| THE HAGUE | 25-04-1989 | SKELLY J.M. |